# FASCICULE DE BREVET EUROPEEN

(11) **EP 1 721 573 B1**
(45) Date de publication et mention de la délivrance du brevet: **13.01.2010**
(21) Numéro de dépôt: 06354013.2
(22) Date de dépôt: 28.04.2006
(51) Int. Cl.: A61B 5/11, G06F 19/00

(54) **Procédé d'estimation de la phase d'un mouvement d'un objet**
Verfahren zur Bewertung der Bewegungsphase eines Objektes
Method for estimating the phase of movement of an object

(30) Priorité: 09.05.2005 FR 0504637
(43) Date de publication de la demande: 15.11.2006
(73) Titulaire: COMMISSARIAT A L'ENERGIE ATOMIQUE, 75752 Paris Cédex 15 (FR)
(72) Inventeur: Heliot, Rodolphe, 38000 Grenoble (FR); David, Dominique, 38640 Claix (FR); Espiau, Bernard, 38190 Sainte Agnes (FR); Pissard-Gibollet, Roger, 38660 Le Touvet (FR)
(74) Mandataire: Hecké, Gérard

(56) Documents cités:
- US-A1- 2004 258 154

## Description

### Domaine technique de l'invention

L'invention concerne un procédé d'estimation de la phase d'un mouvement d'un objet comportant l'acquisition de données expérimentales à partir de mesures de grandeurs physiques par l'intermédiaire d'au moins un capteur associé à l'objet, le procédé comportant une première estimation fiable d'une première plage de valeurs avec une première méthode.

### État de la technique

Pour déterminer la phase d'un mouvement périodique d'un corps à partir de mesures physiques, une technique connue consiste à découper la période en plusieurs zones et à identifier les différentes zones du mouvement par comparaison avec un modèle de référence. En général, les données de plusieurs capteurs sont utilisées pour vérifier une hypothèse de mouvement élaborée à partir de différents modèles.

Le document US 2004/0258154 décrit une méthode d'estimation de vecteurs de mouvement effectuée en plusieurs étapes, les étapes successives utilisant des méthodes de complexité croissante. Une étape postérieure n'est déclenchée que si le résultat de l'étape actuelle est considéré comme insatisfaisant, c'est-à-dire si sa fiabilité est insuffisante. Pour cela, à chaque étape, un indicateur de fiabilité, associé aux vecteurs de mouvement considérés, est calculé, par exemple en utilisant la somme des différences absolues. Cet indicateur de fiabilité est comparé à un seuil, qui tient compte de la fiabilité des vecteurs de mouvement voisins considérés comme satisfaisants dans les étapes précédentes.

Cependant, les techniques connues ne permettent pas d'obtenir une précision satisfaisante en un temps acceptable, notamment dans le cas de détermination de mouvements de prothèses. Les techniques actuelles ne permettent pas de calculer la phase en temps réel, c'est-à-dire au fur et à mesure du déroulé du mouvement, avec un temps de latence faible par rapport au mouvement.

La détermination de la phase du mouvement permet de caractériser différentes postures, par exemple la marche, la course, être assis ou être debout. Ceci peut servir, par exemple, pour la prévention à la chute ou pour analyser un geste sportif pour en corriger les défauts.

Ainsi sont bien connus des appareils comportant des gyromètres positionnés sur les membres inférieurs ou sur le tronc de l'individu. Les méthodes utilisées sont par exemple basées sur la transformée des ondelettes ou sur l'intercorrélation.

### Objet de l'invention

L'invention a pour but de remédier à ces inconvénients et, en particulier, de proposer un procédé permettant d'estimer la phase d'un mouvement avec une meilleure précision et en un temps plus court que les procédés selon l'art antérieur.

Selon l'invention, ce but est atteint par les revendications annexées et, plus particulièrement, par le fait que le procédé comporte au moins une estimation supplémentaire d'une plage supplémentaire de valeurs avec une méthode différente, les méthodes présentant chacune une fiabilité prédéterminée, les plages de valeurs étant observées successivement par ordre de fiabilité décroissante des méthodes correspondantes, chaque plage supplémentaire de valeurs étant comparée avec la plage de valeurs correspondant à la méthode précédente selon ledit ordre, la plage supplémentaire de valeurs étant choisie comme résultat lorsque la plage supplémentaire de valeurs est comprise dans la plage correspondant à une des méthodes précédentes.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
La figure 1 illustre un mode de réalisation particulier du procédé selon l'invention.
La figure 2 illustre des étapes supplémentaires d'un mode de réalisation particulier du procédé selon l'invention.

### Description de modes particuliers de réalisation

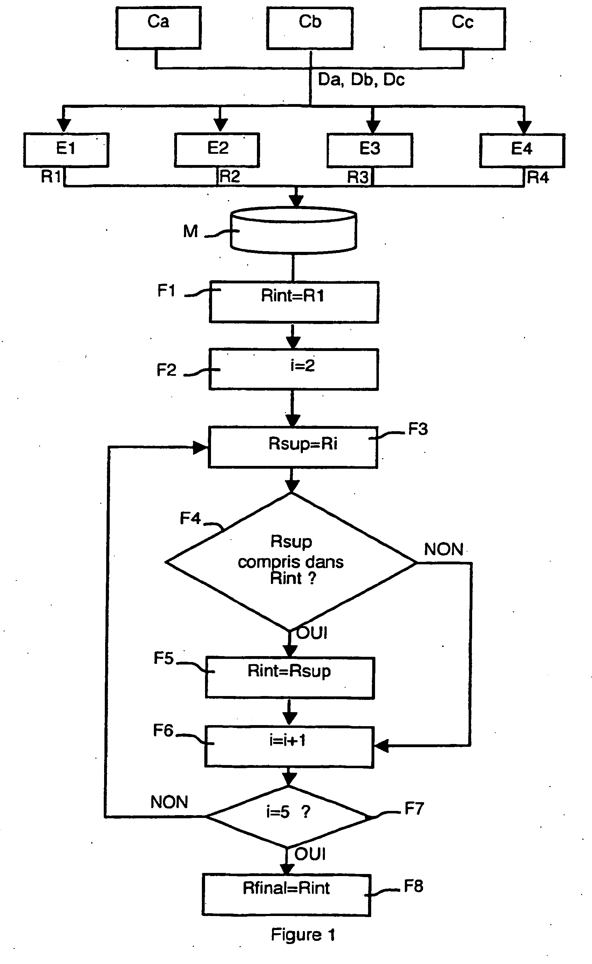

Sur la figure 1, des données expérimentales D (Da, Db, Dc) sont acquises à partir de mesures de grandeurs physiques par l'intermédiaire de plusieurs capteurs C (Ca, Cb, Cc), par exemple des accéléromètres ou des magnétomètres. Les capteurs C sont associés à l'objet, par exemple à un segment articulé, dont la phase de mouvement doit être estimée. On entend par la phase du mouvement la position temporelle dans laquelle on se trouve à un instant donné.

Les capteurs sont, par exemple, reliés à un système d'acquisition portable qui permet également de numériser les signaux analogiques des capteurs et qui comporte un calculateur embarqué pour la détermination de la phase de mouvement.

Les données expérimentales D (Da, Db, Dc) peuvent être au préalable traitées par une transformée de type «chapeau haut de forme» (dite « Top-Hat » en anglais).

Une première plage de valeurs R1 est estimée avec une première méthode par l'intermédiaire d'une première estimation E1 fiable. Des seconde (R2), troisième (R3) et quatrième (R4) plages supplémentaires de valeurs sont estimées respectivement lors de seconde (E2), troisième (E3) et quatrième (E4) estimations supplémentaires. Les estimations peuvent être lancées simultanément et les plages de valeurs R1, R2, R3 et R4 sont, par exemple, stockées dans une mémoire M.

Les quatre estimations sont effectuées avec des méthodes différentes. Les méthodes d'estimation sont caractérisées par deux propriétés principales : leur fiabilité et leur précision. Une méthode est considérée comme fiable lorsque le résultat fourni a une forte probabilité d'être correct. Le résultat d'une méthode correspond habituellement à une plage de valeurs. Plus cette plage est étroite, plus la méthode est considérée comme précise. En général, plus une méthode est fiable, moins elle est précise et vice-versa. La fiabilité peut être déterminée, de manière connue, par exemple de manière empirique. Les méthodes présentent donc chacune une fiabilité prédéterminée et sont classées par ordre de fiabilité décroissant et ainsi, par ordre de précision croissante.

Le procédé selon l'invention permet de gérer les différentes méthodes connues et de les combiner de manière à obtenir un résultat fiable et précis. En particulier, le procédé permet de développer un résultat final en partant de résultats fiables et peu précis et en progressant vers un résultat plus précis qui est tout de même fiable. Ceci est garanti par l'utilisation successive de méthodes de plus en plus précises (donc moins fiables), dont les résultats ne sont retenus que lorsqu'ils sont compatibles avec les résultats fiables des méthodes plus fiables. Ainsi, le risque d'obtenir un résultat final très précis mais peu fiable est écarté, car le résultat final doit toujours être compris dans la plage du résultat au moins de la première méthode, qui est fiable. On raffine donc la précision en conservant la fiabilité initiale.

Ainsi, la première estimation E1 utilise la méthode la plus fiable et donc la moins précise. La seconde estimation E2 utilise la seconde méthode selon l'ordre de la fiabilité. La troisième estimation E3 utilise la troisième méthode selon l'ordre de la fiabilité et la quatrième estimation E4 utilise la méthode la moins fiable.

Les plages de valeurs R sont ensuite observées successivement par ordre de fiabilité décroissante des méthodes correspondantes. Chaque plage supplémentaire (R2, R3, R4) de valeurs est comparée avec la plage de valeurs correspondant à la méthode précédente selon ledit ordre.

Ainsi, sur la figure 1, un résultat intermédiaire Rint prend la valeur de la première plage R1 (fonction F1) et un indice i prend la valeur 2 (fonction F2). Un résultat supplémentaire Rsup prend la valeur de la plage correspondant à l'indice i (fonction F3), donc de la seconde plage R2 à la première exécution de la fonction 3. Ensuite, le résultat intermédiaire Rint est comparé avec le résultat supplémentaire Rsup (fonction F4).

Si le résultat supplémentaire Rsup est compris dans le résultat intermédiaire Rint (sortie OUI de F4), le résultat intermédiaire Rint prend la valeur du résultat supplémentaire Rsup (fonction F5). Le résultat supplémentaire Rsup est ainsi accepté.

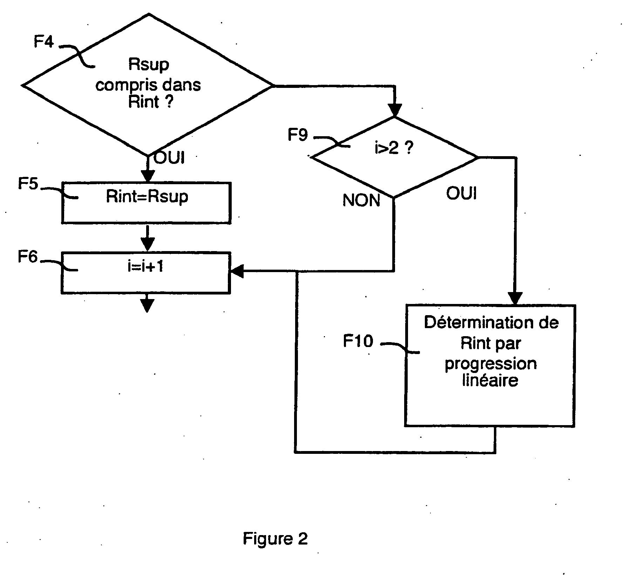

Si le résultat supplémentaire Rsup n'est pas compris dans le résultat intermédiaire Rint (sortie NON de F4), ledit résultat supplémentaire Rsup n'est plus exploité. Le résultat intermédiaire Rint peut, par exemple être conservé, comme représenté à la figure 1. Dans un autre mode de réalisation décrit ci-dessous (figure 2) le résultat intermédiaire Rint peut être remplacé par l'intermédiaire d'une progression linéaire.

Dans les deux cas (OUI ou NON de F4), l'indice i est ensuite incrémenté (fonction F6). Les fonctions F3 à F6 sont répétées, tant que l'indice i est inférieur à une valeur maximale (sortie NON de F7), notamment inférieur à 5 dans le cas d'utilisation de quatre estimations. De manière générale, la valeur maximale de l'indice i pour la fonction F7 est N+1, où N est le nombre de méthodes différentes utilisées.

Lorsque l'indice i est égal à sa valeur maximale (sortie OUI de F7), le résultat final Rfinal prend la valeur du résultat intermédiaire Rint (fonction F8).

Ainsi, lorsqu'une des plages supplémentaires (R2, R3, R4) est comprise dans la plage correspondant à une des méthodes précédentes (R1, R2, R3), la plage supplémentaire (R2, R3, R4) est choisie comme résultat intermédiaire Rint. Le résultat final Rfinal correspond à la valeur que prend le résultat intermédiaire Rint lors de la dernière fois que la fonction F5 est exécutée dans la succession des étapes.

De préférence, on utilise successivement des estimations par une méthode d'analyse qualitative (E1), la méthode par ondelettes (E2), la méthode d'intercorrélation (E3) et la méthode de l'analyse par cyclogramme (E4). Chacune des méthodes est bien connue de l'homme de l'art. Ces méthodes sont basées sur le principe de la comparaison d'un modèle de référence avec des données expérimentales acquises à partir de mesures de grandeurs physiques. Ces méthodes sont, de manière connue, classées selon un ordre de fiabilité décroissante. Toutes ces méthodes fournissent une estimation de la phase.

D'autres méthodes peuvent éventuellement être utilisées, par exemple la comparaison à l'aide d'une somme des carrés des différences ou une méthode utilisant un filtre adaptatif estimant la valeur future de la phase en faisant varier le coefficient de progression de la phase à partir des erreurs mesurées.

L'analyse qualitative est généralement basée sur des graphes d'états et sur l'observation de la dérivée du signal des données, après un filtrage destiné à éliminer le bruit. Cette méthode fournit effectivement une information peu précise, mais elle permet d'obtenir de manière très simple et robuste un premier indice fiable. L'avantage de l'analyse qualitative est qu'elle permet de détecter les phases de démarrage et d'arrêt du mouvement et d'estimer très simplement la cadence à laquelle s'effectue le mouvement. Cette information peut éventuellement être réinjectée dans un calcul utilisant l'intercorrélation. De manière générale, des résultats obtenus avec l'une quelconque des méthodes utilisées peuvent être utilisés dans d'autres méthodes.

La méthode par ondelettes permet de détecter certaines phases du mouvement avec précision et fiabilité, par exemple la pose du talon sur le sol lors de la marche. En effet, à cet instant, un pic d'accélération dû au choc est accompagné d'une hausse significative de l'amplitude des composantes hautes fréquences (par rapport aux fréquences normalement contenues dans le mouvement de la marche) du signal observé. Ainsi, dans ce cas particulier, la méthode par ondelettes fournit une information très précise sur la phase, à un instant donné. Par ailleurs, cette méthode permet de détecter des irrégularités et des discontinuités significatives.

La méthode d'intercorrélation permet d'estimer précisément la phase, mais elle nécessite beaucoup de temps de calcul, ce qui rend difficile, a priori, son exploitation en temps réel. Cependant cette méthode peut être adaptée par l'intermédiaire d'une transformation de type ouverture, ce qui permet d'extraire et d'éliminer des variations lentes du signal et d'amplifier des contrastes.

La méthode de l'analyse par cyclogramme a l'avantage de nécessiter très peu de temps de calcul et permet d'estimer rapidement la phase mais cette méthode est soumise au bruit.

Certaines méthodes peuvent donner, à certains moments, un résultat fiable et très précis à la fois. Un tel résultat peut être accepté automatiquement.

Dans le cas d'un mouvement cyclique, par exemple la marche ou la nage, la phase du mouvement est elle-même périodique. On peut alors l'exprimer en termes de pourcentage dans le cycle du mouvement. Dans ce cas, l'observation peut porter sur le cycle en cours mais également sur des cycles précédents. En plus de l'apport supplémentaire d'information, cela peut permettre d'utiliser des méthodes d'analyse spécifiques aux mouvements cycliques, notamment par cyclogramme.

Comme illustré à la figure 2, lorsque la plage supplémentaire (R2, R3, R4) qui est en train d'être observée n'est pas comprise dans la plage correspondant à la méthode précédente, la plage supplémentaire de valeurs (R2, R3, R4) peut être remplacée par une plage de valeurs obtenue par progression linéaire à partir des plages de valeurs précédentes. Cette progression linéaire peut tenir compte d'un résultat obtenu pour un temps précédent. Ceci nécessite que l'indice i soit supérieur à 2 (sortie OUI de la fonction F9), c'est-à-dire qu'on dispose de la première plage de valeurs R1 et d'au moins une plage supplémentaire R2 pour pouvoir effectuer la progression linéaire. De plus, cette plage supplémentaire R2 doit elle-même être comprise dans la première plage R1. Ainsi, comme représenté à la figure 2, le résultat intermédiaire Rint prend la valeur du résultat de la progression linéaire (fonction F10). Ensuite, le procédé continue conformément à la figure 1, c'est-à-dire l'indice i est incrémenté (fonction F6).

Dans un mode de réalisation simplifié (non-représenté), le procédé est interrompu lorsque la plage supplémentaire (R2, R3, R4) n'est pas comprise dans la plage correspondant à la méthode précédente (sortie NON de F4). Dans ce cas simplifié, les estimations supplémentaires suivantes ne sont pas exploitées et le dernier résultat intermédiaire Rint est retenu comme résultat final Rfinal (la sortie NON de F4 est directement reliée à la fonction F8).

Le procédé peut être interrompu pour d'autres raisons. Par exemple, lorsque la première estimation E1 permet de déterminer que l'objet est à l'arrêt, le procédé peut être arrêté. Si l'analyse par ondelettes permet de déterminer clairement la phase, par exemple par l'intermédiaire d'un pic important des ondelettes, le procédé peut également être arrêté.

## Revendications

1. Procédé d'estimation de la phase d'un mouvement d'un objet comportant l'acquisition de données expérimentales (D) à partir de mesures de grandeurs physiques par l'intermédiaire d'au moins un capteur (C) associé à l'objet, le procédé comportant une première estimation fiable (E1) d'une première plage de valeurs (R1) avec une première méthode, au moins une estimation supplémentaire (E2, E3, E4) d'une plage supplémentaire de valeurs (R2, R3, R4) avec une méthode différente, les méthode présentant chacune une fiabilité prédéterminée, les plages de valeurs (R1, R2, R3, R4) étant observées successivement par ordre de fiabilité décroissante des méthodes correspondantes, chaque plage supplémentaire de valeurs (R2, R3, R4) étant comparée avec la plage de valeurs (R1, R2, R3) correspondant à la méthode précédente selon ledit ordre, la plage supplémentaire de valeurs (R2, R3, R4) étant choisie comme résultat (Rint) lorsque la plage supplémentaire de valeurs (R2, R3, R4) est comprise dans la plage correspondant à une des méthodes précédentes (R1, R2, R3).

2. Procédé d'estimation selon la revendication 1, **caractérisé en ce qu'**il comporte successivement des estimations (E1, E2, E3, E4) par une méthode d'analyse qualitative (E1), la méthode par ondelettes (E2), la méthode d'intercorrélation (E3) et la méthode de l'analyse par cyclogramme (E4).

3. Procédé d'estimation selon l'une des revendications 1 et 2, **caractérisé en ce que**, lorsque la plage supplémentaire (R2, R3, R4) n'est pas comprise dans la plage (R1, R2, R3) correspondant à la méthode précédente, la plage supplémentaire (R2, R3, R4) est remplacée par une plage obtenue par progression linéaire à partir des plages précédentes (R1, R2, R3).

4. Procédé selon l'une quelconque des revendications 1 à 3, **caractérisé en ce que**, lorsque la plage supplémentaire (R2, R3, R4) n'est pas comprise dans la plage (R1, R2, R3) correspondant à la méthode précédente, le procédé est interrompu.

## Claims

1. Process for estimating the motion phase of an object comprising acquisition of experimental data (D) from measurements of physical quantities by means of at least one sensor (C) associated to the object, the process comprising a first reliable estimation (E1) of a first range of values (R1) with a first method, at least one additional estimation (E2, E3, E4) of an additional range of values (R2, R3, R4) with a different method, the methods each presenting a predetermined reliability, the ranges of values (R1, R2, R3, R4) being successively observed in order of decreasing reliability of the corresponding methods, each additional range of values (R2, R3, R4) being compared with the range of values (R1, R2, R3) corresponding to the previous method according to said order, the additional range of values (R2, R3, R4) being chosen as result (Rint) when the additional range of values (R2, R3, R4) is comprised in the range (R1, R2, R3) corresponding to one of the previous methods.

2. Estimation process according to claim 1, **characterized in that** it successively comprises estimations (E1, E2, E3, E4) by a qualitative analysis method (E1), the wavelets method (E2), the intercorrelation method (E3) and the cyclogram analysis method (E4).

3. Estimation process according to one of claims 1 and 2, **characterized in that**, when the additional range (R2, R3, R4) is not comprised in the range (R1, R2, R3) corresponding to the previous method, the additional range (R2, R3, R4) is replaced by a range obtained by linear progression from the previous ranges (R1, R2, R3).

4. Estimation process according to any one of claims 1 to 3, **characterized in that**, when the additional range (R2, R3, R4) is not comprised in the range (R1, R2, R3) corresponding to the previous method, the process is interrupted.

## Patentansprüche

1. Verfahren zur Ermittlung der Bewegungsphase eines Gegenstandes, das die Erfassung von Versuchsdaten (D) ausgehend von Messungen physikalischer Größen mittels mindestens eines dem Gegenstand zugeordneten Messfühlers (C) umfasst, wobei das Verfahren eine erste zuverlässige Ermittlung (E1) eines ersten Wertebereichs (R1) mit einer ersten Methode, mindestens eine zusätzliche Ermittlung (E2, E3, E4) eines zusätzlichen Wertebereichs (R2, R3, R4) mit einer anderen Methode umfasst, wobei die Methoden jeweils eine vorbestimmte Zuverlässigkeit aufweisen, wobei die Wertebereiche (R1, R2, R3, R4) nacheinander beobachtet werden, und zwar in abnehmender Zuverlässigkeitsreihenfolge der entsprechenden Methoden, wobei jeder zusätzliche Wertebereich (R2, R3, R4) mit dem Wertebereich (R1, R2, R3) verglichen wird, der der vorhergehenden Methode entsprechend dieser Reihenfolge entspricht, wobei der zusätzliche Wertebereich (R2, R3, R4) als Ergebnis (Rint) gewählt wird, wenn der zusätzliche Wertebereich (R2, R3, R4) in dem Bereich liegt, der einer der vorhergehenden Methoden (R1, R2, R3) entspricht.

2. Ermittlungsverfahren nach Anspruch 1, **dadurch gekennzeichnet, dass** es nacheinander Ermittlungen (E1, E2, E3, E4) mittels einer Methode der qualitativen Analyse (E1), der Wavelet-Methode (E2), der Interkorrelationsmethode (E3) und der Analyse mittels Zyklogramm-Analyse (E4) umfasst.

3. Ermittlungsverfahren nach einem der Ansprüche 1 und 2, **dadurch gekennzeichnet, dass**, wenn der zusätzliche Bereich (R2, R3, R4) nicht in dem Bereich (R1, R2, R3) liegt, der der vorhergehenden Methode entspricht, der zusätzliche Bereich (R2, R3, R4) durch einen Bereich ersetzt wird, der durch lineare Progression ausgehend von den vorhergehenden Bereichen (R1, R2, R3) erhalten wurde.

4. Verfahren nach einem der Ansprüche 1 bis 3, **dadurch gekennzeichnet, dass**, wenn der zusätzliche Bereich (R2, R3, R4) nicht in dem Bereich (R1, R2, R3) liegt, der der vorhergehenden Methode entspricht, das Verfahren abgebrochen wird.
